# EUROPEAN PATENT APPLICATION

(11) **EP 1 952 872 A1**
(43) Date of publication of application: **06.08.2008**
(21) Application number: 06833000.0
(22) Date of filing: 21.11.2006
(51) Int. Cl.: B01D 46/00, A61L 9/00, A61L 9/01, A61L 9/16, B01D 53/04, B01D 53/86, B01J 20/12, B01J 20/26

(54) **FLUID PURIFYING APPARATUS AND FILTER HOUSING**

(30) Priority: 24.11.2005 JP 2005338623
(71) Applicant: Bridgestone Corporation, Tokyo 104-8340 (JP)
(72) Inventor: MORI, Hisashi, Yokohama-shi Kanagawa 244-0812 (JP)
(74) Representative: Whalley, Kevin
(86) International application number: PCT/JP2006/323150
(87) International publication number: WO 2007/060923

(57) **Abstract**

Disclosed is a fluid purifying apparatus (1) which is composed of a filter housing (2) having a fluid inlet opening (231) and a fluid outlet opening (221), and a filter member (3) contained in the filter housing (2) in such a manner that the filter member crosses the flow path of a fluid from the fluid inlet opening (231) to the fluid outlet opening (221). The fluid purifying apparatus (1) is **characterized in that** the bore diameter a of the fluid outlet opening and the shortest distance b between the filter member and the position of the inner peripheral portion of the fluid outlet opening farthest from the filter member satisfy the following relation: b ≥ a.

## Description

### TECHNICAL FIELD

The present invention relates to a fluid purifying apparatus used to purify a fluid such as gas and liquid.
More particularly, the invention relates to a fluid purifying apparatus of a fluid suction introduction system and/or a fluid pressurization introduction system wherein the pressure loss is reduced as much as possible, and a filter housing for realizing such a fluid purifying apparatus.

### BACKGROUND ART

As a means for removing impurities and odoriferous components contained in a fluid (gas, liquid, etc.), there is generally used a method based on the use of a filter in which a container (filter housing) is packed with a filter medium such as an adsorbent, a composite body having an adsorbent adhered to a substrate, a hollow fiber membrane, a nonwoven fabric, etc. (hereinafter, these are generically referred to as "filter member"), either singly or in combination of two or more of them (refer to, for example, Japanese Patent Laid-open No. Hei 09-070510 and Japanese Patent Laid-open No. 2004-322027). In such a filter, it is desirable that the pressure loss value is reduced as much as possible, from the viewpoint of minimizing the limitations to a pump or blower for moving the fluid, the piping method and the like factors.

Here, the pressure loss value of the filter is lowered if the fill (packing amount) of the adsorbent and filter material with which the filter housing is packed is reduced, but this approach leads to a lowering in the filter capability. Thus, there has been a demand for development of a filter such that both a high adsorption performance or filtering performance of the filter and a low pressure loss of the filter can simultaneously be realized as securely as possible.

Patent Document 1:
   Japanese Patent Laid-open No. Hei 09-070510
Patent Document 2:
   Japanese Patent Laid-open No. 2004-322027

### DISCLOSURE OF THE INVENTION

### Problems to be Solved by the Invention

It is an object of the present invention to provide a fluid purifying apparatus, and a filter housing, with which both a high adsorption performance or filtering performance of the filter and a low pressure loss of the filter can be realized simultaneously and in a high extent.

### Means for Solving the Problems

The present inventor found out that, even where the bore diameters of a fluid inlet opening and a fluid outlet opening of a filter housing are the same and the material and amount of the filter member with which the filter housing is packed are the same, the pressure loss of the filter varies widely depending on the method of packing the filter housing with the filter member. Then, the present inventor made investigations on the method for minimizing the pressure loss due to filter structural factors newly generated upon packing the filter housing with the filter member. As a result of the investigations, it was found out that the pressure loss due to the filter structural factors can be reduced as securely as possible, by providing certain spaces according to the bore diameters of the fluid inlet opening and the fluid outlet opening, between the filter member (with which the filter housing is packed) and the fluid inlet opening and the fluid outlet opening of the filter housing. Based on these findings, the present invention has been completed.

More specifically, the present inventor provides the following fluid purifying apparatuses and filter housings.
Claim 1:
   A fluid purifying apparatus comprising a filter housing having a fluid inlet opening and a fluid outlet opening, and a filter member contained in the filter housing in such a manner that the filter member crosses the flow path of a fluid from the fluid inlet opening to the fluid outlet opening,
   wherein the bore diameter a of the fluid outlet opening and the shortest distance b between the filter member and the position of an inner peripheral portion of the fluid outlet opening farthest from the filter member satisfy the relation: b ≥ a.
Claim 2:
   The fluid purifying apparatus as set forth in claim 1, wherein the area S_{fu} of an end face on the inlet opening side of the filter member and the opening area Sᵤ of the fluid inlet opening satisfy the relation: S_{fu} ≥ Sᵤ.
Claim 3:
   The fluid purifying apparatus as set forth in claim 1 or 2, wherein the bore diameter c of the fluid inlet opening and the shortest distance d between the filter member and the position of an inner peripheral portion of the fluid inlet opening farthest from the filter member satisfy the relation: d ≥ c.
Claim 4:
   The fluid purifying apparatus as set forth in claim 1, 2 or 3, wherein the area S_{fd} of an end face on the outlet opening side of the filter member and the opening area S_{d} of the fluid outlet opening satisfy the relation: S_{fd} ≥ S_{d}.
Claim 5:
   A fluid purifying apparatus comprising a filter housing having a fluid inlet opening and a fluid outlet opening, and a filter member contained in the filter housing in such a manner that the filter member crosses the flow path of a fluid from the fluid inlet opening to the fluid outlet opening,
   wherein the bore diameter c of the fluid inlet opening and the shortest distance d between the filter member and the position of an inner peripheral portion of the fluid inlet opening farthest from the filter member satisfy the relation: d ≥ c.
Claim 6:
   The fluid purifying apparatus as set forth in claim 5, wherein the area S_{fu} of an end face on the inlet opening side of the filter member and the opening area Sᵤ of the fluid inlet opening satisfy the relation: S_{fu} ≥ Sᵤ.
Claim 7:
   The fluid purifying apparatus as set forth in claim 5 or 6, wherein the bore diameter a of the fluid outlet opening and the shortest distance b between the filter member and the position of an inner peripheral portion of the fluid outlet opening satisfy the relation: b ≥ a.
Claim 8:
   The fluid purifying apparatus as set forth in any one of claims 1 to 7, wherein the filter member comprises a fluid purifying adsorbent having a crushed form, a pellet-like shape, or a spherical shape.
Claim 9:
   The fluid purifying apparatus as set forth in claim 8, wherein the fluid purifying adsorbent is at least one selected from the group consisting of coconut shell active carbon, ligneous active carbon, petroleum pitch spherical active carbon, pellet-like molded active carbon, natural zeolite, synthetic zeolite, active clay, surfactant, cation exchange resin, anion exchange resin, cation exchange fiber, anion exchange fiber, chelate resin, chelate compound, inorganic cation adsorbent, inorganic anion adsorbent, inorganic synthetic chemical deodorant, porous adsorbent carrying thereon a compound for chemically decomposing and removing an object gas component by utilizing a chemical reaction such as neutralization, porous adsorbent carrying thereon an oxidation or reduction catalyst comprised of a noble metal or a base metal, and porous adsorbent carrying thereon or coated with a photo-excitation catalyst.
Claim 10:
   The fluid purifying apparatus as set forth in claim 8 or 9, wherein the filter member comprises a filter base material having a three-dimensional network skeleton structure, and at least part of the fluid purifying adsorbent is fixed in the state of coating the three-dimensional network skeleton of the filter base material.
Claim 11:
   The fluid purifying apparatus as set forth in claim 10, wherein the filter base material and the fluid purifying adsorbent are contained in a fluid-permeable cartridge.
Claim 12:
   The fluid purifying apparatus as set forth in any one of claims 1 to 11, wherein the filter housing comprises a nonwoven fabric, a filter paper, a net-like body, or an electrified nonwoven fabric for the purpose of preventing the fluid purifying adsorbent from scattering or for the purpose of removing dust contained in a fluid supplied.
Claim 13:
   A filter housing which has a fluid inlet opening and a fluid outlet opening and which contains a filter member crossing the flow path of a fluid from the fluid inlet opening to the fluid outlet opening,
   wherein the filter housing is formed to be able to contain the filter member in such a manner that the bore diameter a of the fluid outlet opening and the shortest distance b between the filter member and the position of an inner peripheral portion of the fluid outlet opening farthest from the filter member satisfy the relation: b ≥ a.
Claim 14:
   The filter housing as set forth in claim 13, wherein the area S_{fu} of an end face on the inlet opening side of the filter member and the opening area Sᵤ of the fluid inlet opening satisfy the relation: S_{fu} ≥ Sᵤ.
Claim 15:
   The filter housing as set forth in claim 13 or 14, wherein the bore diameter c of the fluid inlet opening and the shortest distance d between the filter member and the position of an inner peripheral portion of the fluid inlet opening farthest from the filter member satisfy the relation: d ≥ c.
Claim 16:
   The filter housing as set forth in claim 13, 14 or 15, wherein the area S_{fd} of an end face on the outlet opening side of the filter member and the opening area S_{d} of the fluid outlet opening satisfy the relation: S_{fd} ≥ S_{d}.
Claim 17:
   A filter housing which comprises a fluid inlet opening and a fluid outlet opening and which contains a filter member crossing the flow path of a fluid from the fluid inlet opening to the fluid outlet opening,
   wherein the filter housing is formed to be able to contain the filter member in such a manner that the bore diameter c of the fluid inlet opening and the shortest distance d between the filter member and the position of an inner peripheral portion of the fluid inlet opening farthest from the filter member satisfy the relation: d ≥ c.
Claim 18:
   The filter housing as set forth in claim 17, wherein the area S_{fu} of an end face on the inlet opening side of the filter member and the opening area Sᵤ of the fluid inlet opening satisfy the relation: S_{fu} ≥ Sᵤ.
Claim 19:
   The filter housing as set forth in claim 17 or 18, wherein the bore diameter a of the fluid outlet opening and the shortest distance b between the filter member and the position of an inner peripheral portion of the fluid outlet opening satisfy the relation: b ≥ a.
Claim 20:
   The filter housing as set forth in any one of claims 13 to 19, wherein the filter member comprises a fluid purifying adsorbent having a crushed form, a pellet-like shape, or a spherical shape, and at least part of the fluid purifying adsorbent is fixed in the state of coating the three-dimensional network skeleton of the filter base material.
Claim 21:
   The filter housing as set forth in claim 20, wherein the filter base material and the fluid purifying adsorbent are contained in a fluid-permeable cartridge.

### Effects of the Invention

The fluid purifying apparatus according to the present invention simultaneously realizes both a high adsorption performance or filtering performance of the filter and a low pressure loss of the filter in a high extent. The fluid purifying apparatus of the present invention is suitably applicable not only to general use for purifying air and water but also to purification of raw material gases supplied to fuel cells, purification of humidifying circulatory water for solid polymer membranes, and air purification and water purification for business use, home use, and vehicle use.
In addition, the filter housing according to the present invention is a filter housing to be used to form the fluid purifying apparatus.

### BRIEF DESCRIPTION OF THE DRAWINGS

FIG. 1 is a longitudinal sectional view of a fluid purifying apparatus showing an embodiment of the present invention.
FIG. 2 is a longitudinal sectional view of a fluid purifying apparatus showing another embodiment of the present invention.
FIG. 3 is a longitudinal sectional view of a fluid purifying apparatus showing a further embodiment of the present invention.

### BEST MODE FOR CARRYING OUT THE INVENTION

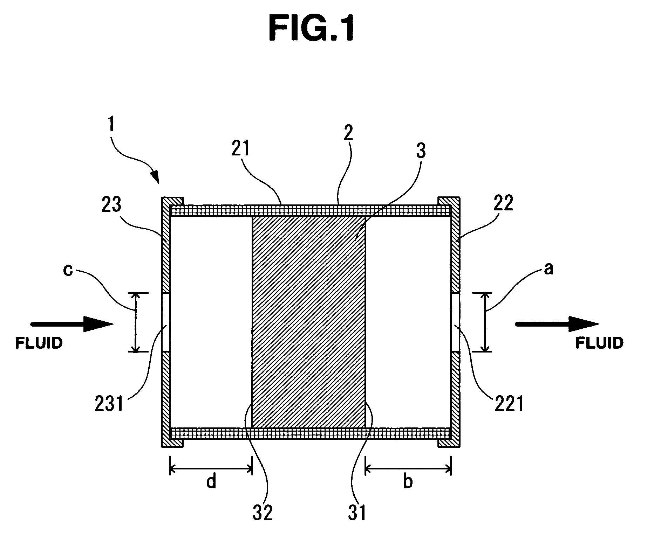

Now, the present invention will be described more in detail below, referring to the drawings. FIG. 1 is a longitudinal sectional view of a fluid purifying apparatus showing an embodiment of the present invention. The fluid purifying apparatus 1 is a fluid purifying apparatus which includes: a filter housing 2 including a cylindrical body 21 for passing a fluid therethrough from the upstream side toward the downstream side, an outlet opening side cover plate 22 having a circular fluid outlet opening 221 and disposed to cover a downstream-side opening of the cylindrical body 21, and an inlet opening side cover plate 23 having a circular fluid inlet port 231 and disposed to cover an upstream-side opening of the cylindrical body 21; and a filter member 3 contained in the filter housing 2.

As shown in FIG. 1, the bore diameter a of the fluid outlet opening 221 and the distance b between the filter member 3 and the position of an inner peripheral portion of the fluid outlet opening 221 farthest from the filter member 3 measured along the axis of the cylindrical body 21 (the shortest distance between the filter member 3 and the position of the inner peripheral portion of the fluid outlet opening 221 farthest from the filter member 3) satisfy the relation: b ≥ a. In addition, the bore diameter c of the fluid inlet opening 231 and the distance d between the filter member 3 and the position of an inner peripheral portion of the fluid inlet opening 231 farthest from the filter member 3 measured along the axis of the cylindrical body 21 (the shortest distance between the filter member 3 and the position of the inner peripheral portion of the fluid inlet opening 231 farthest from the filter member 3) satisfy the relation: d ≥ c.

Here, the expression "fluid outlet opening 221" or "fluid inlet opening 231" means an opening for discharging or introducing a fluid.
In addition, the expression "the position of the inner peripheral portion of the fluid outlet opening 221 farthest from the filter member 3" or "the position of the inner peripheral portion of the fluid inlet opening 231 farthest from the filter member 3" means the position opposed to that position of the inner peripheral portion of the opening for discharging or introducing the fluid which is nearest to the filter member. In other words, the shortest distance between the filter member and the position of the outer peripheral portion of the fluid outlet opening or fluid inlet opening corresponds to the distance obtained by adding the inner diameter of the fluid outlet opening or fluid inlet opening to the shortest distance between the filter member and the inner peripheral portion of the fluid outlet opening or fluid inlet opening.

Since the fluid purifying apparatus according to the present invention satisfies the relation of b ≥ a, it is possible, with respect to the pressure loss measured actually, to minimize the pressure loss due to the new structural factors generated upon packing the filter housing with the filter member.
Here, it is desirable that (the maximum inner diameter of the cylindrical body) ≥ a > 0, and that (the length of the cylindrical body) × (1/2) ≥ b > 0. If the values of a and b fall out of these relations, the structural pressure loss may be enlarged upon packing the housing with the adsorbent and the filter member.

On the other hand, since the fluid purifying apparatus 1 according to the present invention satisfies the relation of d ≥ c, it is possible, with respect to the pressure loss measured actually, to minimize the pressure loss due to the new structural factors generated upon packing the filter housing with the filter member.
Here, it is desirable that (the maximum inner diameter of the cylindrical body) ≥ c > 0, and that (the length of the cylindrical body) × (1/2) ≥ d > 0. If the values of c and d fall out of these relations, the structural pressure loss may be enlarged upon packing the housing with the adsorbent and the filter member.

Incidentally, the adoption of the configuration of b ≥ a or the configuration of d ≥ c in designing the fluid purifying apparatus has not usually been made by those skilled in the art, since such a configuration tends to reduce the fill (packing amount) of the filter member. However, the present inventor found out such a configuration to be an effective configuration from the viewpoint of minimizing the pressure loss arising from the structural factors of the filter as above-mentioned.

In the fluid purifying apparatus 1, the filter member 3 is packingly disposed so as to close the inside of the cylindrical body 21 (so as to close, while crossing, the flow path of a fluid from the fluid inlet opening 231 to the fluid outlet opening 221), and has an outlet opening side end face 31 facing the fluid outlet opening 221 and an inlet opening side end face 32 facing the fluid inlet opening 231.
Besides, the fluid purifying apparatus 1 is so configured that the area S_{fd} of the outlet opening side end face 31 and the opening area S_{d} of the fluid outlet opening 221 satisfy the relation of S_{fd} ≥ S_{d}, and is so configured that the area S_{fu} of the inlet opening side end face 32 and the opening area Sᵤ of the fluid inlet opening 231 satisfy the relation of S_{fu} ≥ Sᵤ.

With the fluid purifying apparatus 1 so configured as to satisfy the relation of S_{fd} ≥ S_{d} and/or the relation of S_{fu} ≥ Sᵤ, this system is applicable also to the case where a reduction in cross-sectional area is needed in connecting the fluid purifying apparatus and a fluid drive source such as a suction/pressurizing pump.
Incidentally, where the values of S_{fu} and Sᵤ fall out of these relations, or where the values of S_{fd} and S_{d} fall out of these relations, the pressure loss may be increased extremely.

Here, in the case where the area Sᵤ of the fluid inlet opening is smaller than the area S_{fu} of the inlet opening side end face of the filter member and where the spatial distance (the value of d) between the filter member 3 and the fluid inlet opening 231 is smaller than the bore diameter (the value of c) of the fluid inlet opening 231, an abrupt change is generated in the manner of flow of the fluid in the vicinity of the fluid inlet opening 231, so that the pressure loss is increased, and a portion of the filter member or the adsorbent packing the filter housing may fail to act effectively. This naturally is undesirable.

In the present invention, the materials for forming the cylindrical body 21, the outlet opening side cover plate 22, and the inlet opening side cover plate 23 constituting the filter housing 2 are not particularly limited to metals, resins or the like. However, it is desirable that the materials are capable of enduring pressure variations at the time of introducing the fluid and are little changed in dimensions during use.

On the other hand, the filter member 3 with which the filter housing 2 is packed can be appropriately selected according to the physical properties of the fluid applied. From the viewpoint of maximal exhibition of the internal filtering function of the filter, however, use is made of a filter member having an adsorbent supported on a filter base material having a three-dimensional network structure, a filter member having an adsorbent supported on a honeycomb filter base material, or a fluid purifying adsorbent in a crushed form, a pellet-like shape or a spherical shape.

Examples of the fluid purifying adsorbent which can be used, from the viewpoint of removing impurities according to the purpose of use, include: coconut shell active carbon, ligneous active carbon, petroleum pitch spherical active carbon, pellet-like molded active carbon, natural zeolite, synthetic zeolite, active clay, surfactant, cation exchange resin, anion exchange resin, cation exchange fiber, anion exchange fiber, chelate resin, chelate compound, inorganic cation adsorbent, inorganic anion adsorbent, inorganic synthetic chemical deodorant, porous adsorbent carrying thereon a compound for chemically decomposing and removing an object gas component by utilizing a chemical reaction such as neutralization, porous adsorbent carrying thereon an oxidation or reduction catalyst comprised of a noble metal or a base metal, and porous adsorbent carrying thereon or coated with a photo-excitation catalyst such as titanium oxide and the like.

In addition, as the filter member 3 in the present invention, a filter member which includes a filter base material having a three-dimensional network skeleton structure and in which at least a portion of the fluid purifying adsorbent is fixed in the state of coating the three-dimensional network skeleton of the filter base material is used preferably, from the viewpoint of easily and assuredly performing the operation of containing the filter member while securing a certain space inside the filter housing.

Incidentally, as the filter member 3, a filter member contained in a fluid-permeable (gas-permeable or liquid-permeable) cartridge is used preferably, from the viewpoints of handleability, workability, and maintainability.

Furthermore, it is preferable, for the purpose of preventing the fluid purifying adsorbent from scattering or for the purpose of removing dust contained in the fluid supplied, that the filter housing 2 has a nonwoven fabric, a filter paper, a net-like body, or an electrified nonwoven fabric. Examples of the nonwoven fabric, the filter paper or the net-like body include electrified polypropylene-made nonwoven fabric or HEPA, ULPA and the like filter members. It suffices that such a filter member is disposed on the inlet opening side and/or the outlet opening side of the housing.

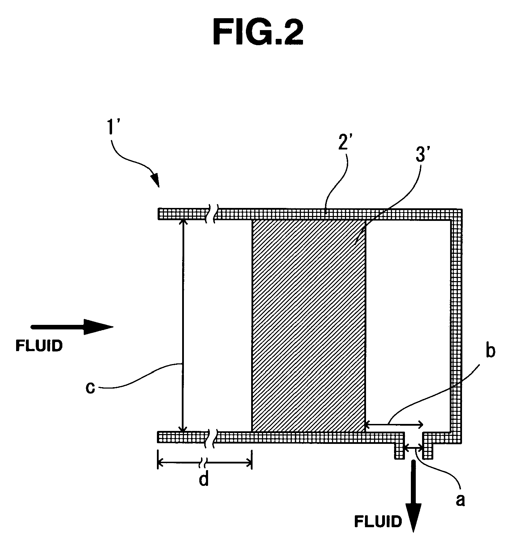

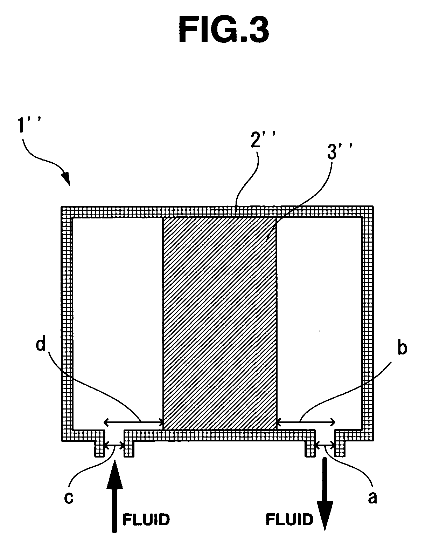

While the cylindrical body 21, the outlet opening side cover plate 22, and the inlet opening side cover plate 23 are separate bodies in the above-described fluid purifying apparatus 1, they may be molded or formed integrally. The layout positions of the fluid inlet opening 231 and the fluid outlet opening 221 are not particularly limited, and these openings may be laid out at the cylindrical body 21 or at positions corresponding to the cylindrical body 21 (FIGS. 2 and 3 show longitudinal sectional views of a fluid purifying apparatus 1' representing another embodiment of the present invention and a fluid purifying apparatus 1" representing a further embodiment of the invention).
Incidentally, in these apparatuses 1' and 1", symbols 2' and 2" denote filter housings, symbols 3' and 3" denote filter members, and symbols a, b, c, d have the same meanings as above, respectively.
In addition, while the filter member 3 has the flat end faces 31, 32, the end faces may be rugged (may have projections and recesses). While the end faces 31, 32 are formed to be perpendicular to the axis of the cylindrical body 21, the end faces may each be formed at an inclination against the axis of the cylindrical body.
Furthermore, while the cylindrical body 21, the fluid outlet opening 221 and the fluid inlet opening 231 are circular in cross-sectional shape with reference to the axis of the cylindrical body 21, these cross-sectional shapes are not particularly limited; the cross-sectional shapes may each be a polygon such as tetragon, pentagon, etc. or an ellipse. In the cases where the cross-sectional shapes are not circular in plane view, the term "bore diameter" used in the present invention means a "minimum inner diameter".

### EXAMPLES

Now, the present invention will be described specifically by showing Production Example, Examples, and Comparative Examples, but the invention is not to be limited to the following examples.

### Production Example

A polyurethane foam having a three-dimensional network skeleton structure (product code: HR-10, produced by Bridgestone Corporation; 5 mm thick) was impregnated with an acrylic emulsion binder having a solid content of 50% (product code: E-1054-6, produced by Soken Chemical & Engineering Co., Ltd.) so as to obtain a solid content of 25 g/L, followed by drying at 100°C for 5 min. Then, coconut shell active carbon having a mean particle diameter of 30 mesh was deposited on the polyurethane foam from the face and back sides, and surplus of active carbon was shaken off, to obtain an active carbon filter sheet having an active carbon deposition amount of 150±20 g/L.
The active carbon filter sheet was blanked into 67 mmφ disks, and 26 sheets of the disks were stacked, to obtain a filter (overall thickness: 130 mm).

### Examples 1-3, Comparative Examples 1, 2

Under the conditions as shown in Table 1 below, the above-mentioned filter member was placed in a fluid suction introduction type filter housing (cylindrical shape of inner diameter 67 mmφ x packing height 175 mm; a fluid inlet opening and a fluid outlet opening are circular in plane-view shape; the normal line passing through the centers of the circular shapes coincides with the axis of the filter housing), to obtain a fluid purifying apparatus, and the pressure loss of the apparatus was evaluated. The results are shown in Table 1 below.

**Table 1**

| | | Bore diameter | | Distance | | Pressure loss |
|---|---|---|---|---|---|---|
| | | Suction side | Discharge side | Suction side | Discharge side | |
| | | c (mm) | a (mm) | d (mm) | b (mm) | (Pa) |
| Example | 1 | 67 | 5 | open | 5 | 975 |
| | 2 | 67 | 5 | open | 10 | 975 |
| | 3 | 67 | 5 | open | 15 | 975 |
| Comparative Example | 1 | 67 | 5 | open | 0 | 1825 |
| | 2 | 67 | 5 | open | 3 | 1025 |

### Bore diameter

Bore diameters of the fluid inlet opening and the fluid outlet opening. The bore diameter being 67 mmφ means that the flow path inside wall surface of the fluid inlet opening or the fluid outlet opening is formed to be steplessly continuous with the inside wall of the filter housing.

### Distance

The distance from the center position of the fluid inlet opening or the center position of the fluid outlet opening to the end face of the filter member, measured along the axis of the filter housing.

### Suction side, Discharge side

The "suction side" means the side on which the fluid inlet opening is disposed, and the "discharge side" means the side on which the fluid outlet opening is disposed.

### Pressure loss

A suction pump was connected to the fluid outlet opening side (downstream side) of the filter housing containing the filter, and a flow meter and a pressure gauge were disposed between the filter housing and the suction pump, to construct a pressure loss measuring apparatus. First, the filter housing containing the filter as the object of measurement was connected, and, in this condition, air was sucked in at a suction flow rate of 50 L/min, and the pressure loss in this situation was measured. Incidentally, the whole periphery of the housing inside wall on the fluid inlet opening side was taped with a rubber tape so as to prevent the active carbon filter in the packing state from dropping off, and the filter housing was provided in its upper portion with an exhaust opening with a bore diameter of 5 mmφ so that air purified by the active carbon filter could be obtained through the exhaust opening.
Next, a blank pressure loss was measured in the same conditions as above, except that the filter housing containing the filter was detached. By subtracting the blank pressure loss from the above-mentioned pressure loss, the "pressure loss" value in Table 1 was calculated.

### Examples 4-6, Comparative Examples 3-5

The pressure loss of each filter was evaluated in the same manner as in the case of Table 1 above, except that the bore diameter on the discharge side was set to 10 mm. The results are shown in Table 2 below.

**Table 2**

| | | Bore diameter | | Distance | | Pressure |
|---|---|---|---|---|---|---|
| | | Suction Side | Discharge side | Suction side | Discharge Side | loss |
| | | c (mm) | a (mm) | d (mm) | b (mm) | (Pa) |
| Example | 4 | 67 | 10 | Open | 10 | 87 |
| | 5 | 67 | 10 | Open | 15 | 87 |
| | 6 | 67 | 10 | Open | 20 | 87 |
| Comparative Example | 3 | 67 | 10 | Open | 0 | 325 |
| | 4 | 67 | 10 | Open | 5 | 165 |
| | 5 | 67 | 10 | Open | 7 | 125 |

### Examples 7-9, Comparative Examples 6-8

The pressure loss of each filter was evaluated in the same manner as in the case of Table 1 above, except that the bore diameter on the discharge side was set to 15 mm. The results are shown in Table 3 below.

**Table 3**

| | | Bore diameter | | Distance | | Pressure loss |
|---|---|---|---|---|---|---|
| | | Suction Side | Discharge side | Suction side | Discharge Side | |
| | | c (mm) | a (mm) | d (mm) | b (mm) | (Pa) |
| Example | 7 | 67 | 15 | Open | 15 | 65 |
| | 8 | 67 | 15 | Open | 20 | 65 |
| | 9 | 67 | 15 | Open | 25 | 65 |
| Comparative Example | 6 | 67 | 15 | Open | 0 | 150 |
| | 7 | 67 | 15 | Open | 7 | 125 |
| | 8 | 67 | 15 | Open | 12 | 100 |

### Examples 10-13, Comparative Example 9

The pressure loss of each filter was evaluated in the same manner as in the case of Table 1 above, except that the bore diameter on the suction side was set to 10 mm.

**Table 4**

| | | Bore diameter | | Distance | | Pressure loss |
|---|---|---|---|---|---|---|
| | | Suction side | Discharge side | Suction side | Discharge Side | |
| | | c (mm) | a (mm) | d (mm) | b (mm) | (Pa) |
| Example | 10 | 10 | 10 | 0 | 10 | 415 |
| | 11 | 10 | 10 | 10 | 10 | 200 |
| | 12 | 10 | 10 | 15 | 10 | 200 |
| | 13 | 10 | 10 | 20 | 10 | 200 |
| Comparative Example | 9 | 10 | 10 | 0 | 0 | 570 |

## Claims

1. A fluid purifying apparatus comprising a filter housing having a fluid inlet opening and a fluid outlet opening, and a filter member contained in said filter housing in such a manner that said filter member crosses the flow path of a fluid from said fluid inlet opening to said fluid outlet opening,
wherein the bore diameter a of said fluid outlet opening and the shortest distance b between said filter member and the position of an inner peripheral portion of said fluid outlet opening farthest from said filter member satisfy the relation: b ≥ a.

2. The fluid purifying apparatus as set forth in claim 1, wherein the area S_{fu} of an end face on the inlet opening side of said filter member and the opening area Sᵤ of said fluid inlet opening satisfy the relation: S_{fu} ≥ Sᵤ.

3. The fluid purifying apparatus as set forth in claim 1 or 2, wherein the bore diameter c of said fluid inlet opening and the shortest distance d between said filter member and the position of an inner peripheral portion of said fluid inlet opening farthest from said filter member satisfy the relation: d ≥ c.

4. The fluid purifying apparatus as set forth in claim 1, 2 or 3, wherein the area S_{fd} of an end face on the outlet opening side of said filter member and the opening area S_{d} of said fluid outlet opening satisfy the relation: S_{fd} ≥ S_{d}.

5. A fluid purifying apparatus comprising a filter housing having a fluid inlet opening and a fluid outlet opening, and a filter member contained in said filter housing in such a manner that said filter member crosses the flow path of a fluid from said fluid inlet opening to said fluid outlet opening,
wherein the bore diameter c of said fluid inlet opening and the shortest distance d between said filter member and the position of an inner peripheral portion of said fluid inlet opening farthest from said filter member satisfy the relation: d ≥ c.

6. The fluid purifying apparatus as set forth in claim 5, wherein the area S_{fu} of an end face on the inlet opening side of said filter member and the opening area Sᵤ of said fluid inlet opening satisfy the relation: S_{fu}≥ Sᵤ·

7. The fluid purifying apparatus as set forth in claim 5 or 6, wherein the bore diameter a of said fluid outlet opening and the shortest distance b between said filter member and the position of an inner peripheral portion of said fluid outlet opening satisfy the relation: b ≥ a.

8. The fluid purifying apparatus as set forth in any one of claims 1 to 7, wherein said filter member comprises a fluid purifying adsorbent having a crushed form, a pellet-like shape, or a spherical shape.

9. The fluid purifying apparatus as set forth in claim 8, wherein said fluid purifying adsorbent is at least one selected from the group consisting of coconut shell active carbon, ligneous active carbon, petroleum pitch spherical active carbon, pellet-like molded active carbon, natural zeolite, synthetic zeolite, active clay, surfactant, cation exchange resin, anion exchange resin, cation exchange fiber, anion exchange fiber, chelate resin, chelate compound, inorganic cation adsorbent, inorganic anion adsorbent, inorganic synthetic chemical deodorant, porous adsorbent carrying thereon a compound for chemically decomposing and removing an object gas component by utilizing a chemical reaction such as neutralization, porous adsorbent carrying thereon an oxidation or reduction catalyst included of a noble metal or a base metal, and porous adsorbent carrying thereon or coated with a photo-excitation catalyst.

10. The fluid purifying apparatus as set forth in claim 8 or 9, wherein said filter member comprises a filter base material having a three-dimensional network skeleton structure, and at least part of said fluid purifying adsorbent is fixed in the state of coating the three-dimensional network skeleton of said filter base material.

11. The fluid purifying apparatus as set forth in claim 10, wherein said filter base material and said fluid purifying adsorbent are contained in a fluid-permeable cartridge.

12. The fluid purifying apparatus as set forth in any one of claims 1 to 11, wherein said filter housing comprises a nonwoven fabric, a filter paper, a net-like body, or an electrified nonwoven fabric for the purpose of preventing said fluid purifying adsorbent from scattering or for the purpose of removing dust contained in a fluid supplied.

13. A filter housing which has a fluid inlet opening and a fluid outlet opening and which contains a filter member crossing the flow path of a fluid from said fluid inlet opening to said fluid outlet opening,
wherein said filter housing is formed to be able to contain said filter member in such a manner that the bore diameter a of said fluid outlet opening and the shortest distance b between said filter member and the position of an inner peripheral portion of said fluid outlet opening farthest from said filter member satisfy the relation: b ≥ a.

14. The filter housing as set forth in claim 13, wherein the area S_{fu} of an end face on the inlet opening side of said filter member and the opening area Sᵤ of said fluid inlet opening satisfy the relation: S_{fu} ≥ Sᵤ.

15. The filter housing as set forth in claim 13 or 14, wherein the bore diameter c of said fluid inlet opening and the shortest distance d between said filter member and the position of an inner peripheral portion of said fluid inlet opening farthest from said filter member satisfy the relation: d ≥ c.

16. The filter housing as set forth in claim 13, 14 or 15, wherein the area S_{fd} of an end face on the outlet opening side of said filter member and the opening area S_{d} of said fluid outlet opening satisfy the relation: S_{fd} ≥ S_{d}.

17. A filter housing which comprises a fluid inlet opening and a fluid outlet opening and which contains a filter member crossing the flow path of a fluid from said fluid inlet opening to said fluid outlet opening,
wherein said filter housing is formed to be able to contain said filter member in such a manner that the bore diameter c of said fluid inlet opening and the shortest distance d between said filter member and the position of an inner peripheral portion of said fluid inlet opening farthest from said filter member satisfy the relation: d ≥ c.

18. The filter housing as set forth in claim 17, wherein the area S_{fu} of an end face on the inlet opening side of said filter member and the opening area Sᵤ of said fluid inlet opening satisfy the relation: S_{fu} ≥ Sᵤ.

19. The filter housing as set forth in claim 17 or 18, wherein the bore diameter a of said fluid outlet opening and the shortest distance b between said filter member and the position of an inner peripheral portion of said fluid outlet opening satisfy the relation: b ≥ a.

20. The filter housing as set forth in any one of claims 13 to 19, wherein said filter member comprises a fluid purifying adsorbent having a crushed form, a pellet-like shape, or a spherical shape, and at least part of said fluid purifying adsorbent is fixed in the state of coating the three-dimensional network skeleton of said filter base material.

21. The filter housing as set forth in claim 20, wherein said filter base material and said fluid purifying adsorbent are contained in a fluid-permeable cartridge.
